# EUROPEAN PATENT APPLICATION

(11) **EP 4 660 311 A1**
(43) Date of publication of application: **10.12.2025**
(21) Application number: 24180234.7
(22) Date of filing: 05.06.2024
(51) Int. Cl.: C12P 7/625, C08G 63/06, C08G 63/78

(54) **NOVEL CO-BIOPOLYMERS**

(71) Applicant: CO2BioClean GmbH, 65760 Eschborn (DE)
(72) Inventor: Fantinel, Fabiana, 61476 Kronberg (DE); Shanmungan, Anusriha, 65933 Frankfurt am Main (DE); Wang, Yunqian, 65795 Hattersheim am Main (DE); Tettenborn, Moritz, 60596 Frankfurt am Main (DE)
(74) Representative: Patentanwälte Gierlich & Pischitzis Partnerschaft mbB

(57) **Abstract**

The present invention discloses a method for producing PHA-copolymer using bacteria, by using a two-step process. In the first step the bacteria are grown under heterotrophic conditions using an organic substance as carbon source and exponential growth conditions. In a second step the bacteria are then cultivated under autotrophic conditions under an atmosphere of H₂, CO₂ and O₂, wherein the O₂ content is less than 10 % (v/v) and the pressure is more than 1 barg and at least one precursor for further monomers is added before and/or during this step, wherein the precursor is selected from an organic substance comprising at least 3 carbon atoms and at least 2 oxygen atoms, wherein the organic substance in its hydrolyzed form comprises at least two of carbon atoms, which are part of a functional group selected from COOH, C=O, C-OH. By this the production of PHA-copolymers with unique properties and at a high rate is possible.

## Description

### FIELD OF THE INVENTION

The present invention relates to a method for producing polyhydroxyalkanoate(PHA)-copolymers using a wild type bacteria and extracting the produced PHA in an efficient way.

PHA is the general term for a range of diverse biodegradable polymers that consist of polyesters of 3-hydroxyalkanoic acids. These polymers are of interest due to a broad range of applications and the fact that they are completely biodegradable thus offering little or no long term waste issues.

PHAs are generally classified as short chain length PHAs (sclPHAs), medium chain length PHAs (mclPHAs) or long chain length PHAs (lclPHAs), depending upon the number of carbon atoms of the constituting monomers thereof. SclPHA comprises monomers of C₃-C₅, mclPHA comprises monomers of C₆-C₁₄, and lcl-PHA comprises monomers of more than 14 carbons (>C₁₄). This variation in monomer chain length gives rise to different properties in the polymer, with sclPHAs and lclPHAs both having different properties, sclPHAs having a high degree of crystallinity and being usually rigid and brittle, and lclPHAs being sticky and very difficult to handle. The properties of sclPHAs and lclPHAs limit the range of their applications. But since sclPHAs are easier obtainable there is need of sclPHAs with improved properties.

PHA structures can vary in two ways. First, PHAs can vary according to the structure of the pendant groups, which are typically attached to a carbon atom having (R)-stereochemistry. The pendant groups form the side chain of hydroxy alkanoic acid not contributing to the PHA-carbon backbone. Second, PHAs can vary according to the number and types of their repeat units. For example, PHAs can be homopolymers, copolymers, or terpolymers. These variations in PHA structure can cause variations in their physical characteristics. These physical characteristics make PHAs useful for a number of products that may be commercially valuable.

The stereochemistry in the monomers may be only R or S, or monomers of both types may be present. This further influences the properties of the polymer.

The several types of PHAs make PHAs a versatile family of polymers with properties tuneable by molecular design. For example, short chain length scl-PHA are divided into P3HB, commonly simply polyhydroxobutyrate (PHB), P4HB, (valeric acid copolymer) PHBV, PHBH, P3HB4HB, medium chain length mcl-PHA are PHBH (hexanoic acid copolymer), PHBO (octanoic acid copolymer) and PHBD (dodecanoic acid copolymer).

The chemical structure of PHAs may be described as a polymeric chain formed by repetitions of the following unit:

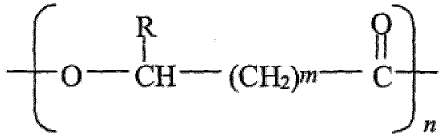

Where R is an alkyl or alkenyl group of variable length and m and n are integers, in some polymers R and m assuming the following values:
PHB: R=CH₃, m=1
PHBV: R=CH₃ or CH₃-CH₂-, m=1
P4HB: R=H, m=2
P3HB4HB: R=H for m=2 or R=CH₃ for m=1

For mclPHA the length of the alkyl chain of R can be different, e.g. for polyhydroxyhexanoate PHBH R is CH₃-CH₂-CH₂- and m equals 2.

PHA-copolymers comprise a mixture of several different monomer units. The term copolymer as used in this application may also comprise polymer with more than two different monomer units, e.g. three (terpolymers) or more.

Due to their structure the PHA monomer units contain a chiral carbon atom. The polymer may therefore comprise monomers differing in their configuration. PHA synthesized by organisms usually only contains monomers in R-configuration due to the enzymatic pathway.

Besides plants and other organisms, bacteria are very useful for producing PHA. In recent years many efforts were taken to use genetically modified or unmodified bacteria (Wild type) in order to produce PHA also at an industrial scale.

Schlegel et al. Nature 1961, 191, 463-465 "Formation and utilization of poly-β-hydroxybutyric acid by Knallgas bacteria (*hydrogenomonas*)*"* found that PHAs, namely PHB can be produced under nutritional stress conditions, especially using an atmosphere comprising CO₂, H₂ and O₂.

Ayaaki Ishizaki and Kenji Tanaka Journal of fermentation and bioengineering 1990, 69(3), 170-174 "Batch Culture of Alcaligenes eutrophus ATCC 17697T using recycled gas closed circuit culture system", Ayaaki Ishizaki and Kenji Tanaka Journal of Fermentation and bioengineering 1991, 71(4) 254-257. "Production of Poly-b-Hydroxybutyric Acid from Carbon Dioxide by Alcaligenes eutrophus ATCC17697T" and Toshihiro Takeshita et al. J. Fac. Agr. Kyushu Univ. 1993, 38(1-2), 55-64. *"*Studies on Dissolved Hydrogen Behavior in Autotrophic Culture of Alcaligenes autrophus ATCC 17697T*"* disclose the synthesis of PHB in bacteria under autotrophic conditions. One drawback of the conditions is the requirement of ratio of hydrogen and oxygen, which is explosive (oxygen > 6 %). This limits the use of these conditions.

Kenji Tanaka and Ayaaki Ishizaki Journal of fermentation and bioengineering, 1994, 77(4), 425-427 *"*Production of Poly-D-3-Hydroxybutyric Acid from Carbon Dioxide by a Two-Stage Culture Method Employing Alcaligenes eutrophus ATCC 17697 T*"* discloses a two-stage heterotrophic-autotrophic growth with fructose and O₂ in autotrophic stage in an amount of 2-3%. But the PHA storage efficiency of bacteria is decreased with organic substrates.

The use of CO₂ as carbon source makes these processes very valuable for the environmentally friendly production of plastics, which are even biodegradable.

US 5,942,597 and WO 97/07229 A1 describe the extraction from PHA from oil plants using solvent mixtures.

The applications WO 2021/130128 A1 and WO 2022/268899 A1 describe the production of PHA and PHA-copolymers using increased pressure.

Among other PHA types, currently mostly PHB is successfully produced on industrial scale by process that make use of glucose as feedstock. PHB obtained by industrial process are highly crystalline due to their chemical structure composed by a single monomeric unit which is optically pure (high stereoregularity). However, introducing chain irregularities from comonomer, or insertion, e.g. 4B vs. 3B or else stereoregularity is very important to achieve improved flexibility and thus better performances and processability for most general plastics applications.

Usually, co-monomers are used to obtain less crystalline polymers, which are usually smoother and more elastic.

The procedures presented in the prior art are not suitable for industrial scale processing. Also, the extraction of the PHA produced from the bacteria is difficult. They also require long reaction times.

Also, the production of copolymers especially with longer chains is difficult and these monomers are only incorporated in a minor amount using biological processes.

### BRIEF SUMMARY OF THE INVENTION

It is, therefore, an object of the present invention to provide a method for producing and preferably further purifying PHA-copolymers in bacteria, more preferably on an industrial scale.

This aim is achieved by the inventions as claimed in the independent claims. Advantageous embodiments are described in the dependent claims.

The object of the invention is also achieved by a method. In what follows, individual steps of a method will be described in more details. The steps do not necessarily have to be performed in the order given in the text. Also, further steps not explicitly stated may be part of the method.

The object of the invention is achieved by a method for producing PHA-copolymers comprising the following steps:
a) growing bacteria under heterotrophic conditions in a media;
b) cultivating the bacteria under autotrophic conditions under an atmosphere of CO₂, H₂ and optional O₂, wherein the amount on O₂ is below 10 % (v/v) and pressure is at least 1 barg, wherein at least one precursor for further monomers is added before and/or during step b), wherein the at least one precursor is selected from an organic substance comprising at least 3 carbon atoms and at least 2 oxygen atoms, wherein the organic substance in its hydrolyzed form comprises at least two of carbon atoms are part of a functional group selected from COOH, C=O, C-OH.

The bacteria that are useful in the present invention include any bacteria that can produce PHAs, preferably bacteria which naturally produce PHAs. Wild type bacteria are preferred. Such bacteria are not genetically engineered. By using a wild type the process has to fulfil less strict regulations.

In one embodiment, the bacterium is *Pelomonas saccharophila* (also formerly known as *Pseudomonas saccharophila*)*, Azomonas lata* (also formerly known as *Alcaligenes latus*) and *R. eutropha* (also known as *Cupriavidus necator*)*.* These are non-pathogenic, gram-negative bacteria, which can be found in soil and water. Their facultative chemolithoautotrophic metabolism allows them to grow either on organic compounds or using H₂ and CO₂ as reductive agent and carbon source, respectively, when submitted to a nutrient limitation and in presence of oxygen. Both modes can also be concomitant depending on the availability of nutrients.

In a preferred embodiment the bacteria are the wild bacteria selected from *Cupriavidus necator*, even more preferably stream *Cupriavidus necator* H16, which is a non-pathogenic, gram-negative stream. Other preferred steams are the streams available under the DSM numbers DSM -428, DSM-531, DSM-11098, DSM-3102, DSM-529 and DSM-545 at the DSMZ-German Collection of Microorganisms and Cell Cultures GmbH.

Additionally, wild bacteria selected from *Preudosomas Putida* or *Aeuruginosa* can be used.

In the first step the bacteria are grown under heterotrophic conditions. These are conditions utilizing organic compounds as carbon and energy source. In a preferred embodiment under these conditions the bacteria show exponential growth.

In a preferred embodiment the bacteria are grown using conditions with no limitations regarding the nutrients, especially nitrogen, carbon or phosphor.

Usually, an ambient atmosphere is used.

In a preferred embodiment the step is performed at ambient pressure or a pressure resulting only from the reaction conditions, e.g. heating in a closed vessel.

The medium used for step a) is an aqueous medium.

In a preferred embodiment the medium for the first step comprises at least one Ammonium salt as nitrogen source, preferably Ammonium sulphate.

As carbon source different organic substances may be used. This may be sugars like sucrose, fructose or glucose, polyols like glycerol, organic acids or salts or esters thereof, as acetic acid or malate or ethyl acetate. The carbon source is preferably soluble in the medium.

In a preferred embodiment the molecular mass of each carbon source used is below 600 g/mol preferably below 500 g/mol, even more preferably below 400 g/mol.

In a preferred embodiment the medium for the first step comprises at least one phosphate salt as phosphor source, preferably ammonium, sodium or potassium salts of phosphates, especially in their monobasic form may be used, more preferably an H₂PO₄ salt, more preferably (NH₄)H₂PO₄, KH₂PO₄ and/or NaH₂PO₄. The salts may be used a hydrate.

Ammonium hydroxide, hydrochloric acid, citric acid and/or sulfuric acid may be used for adjusting the pH.

The medium may comprise further salts and additives, like magnesium salts, iron salts, calcium salts, vitamins, yeast extract or trace elements like Zn, B, Co, Cu, Ni, Mo or Mn.

The pH of the medium is preferably 4.5 to 7.5, more preferably 6.4 to 7.1.

In a preferred embodiment the amount of C at the beginning of step a) is between 2 and 50 g/l, preferably between 5 and 30 g/l.

In a preferred embodiment the amount of N at the beginning of step a) is between 0.1 and 5 g/l, preferably 0.1 and 4 g/l.

In a preferred embodiment the amount of P at the beginning of step a) is between 0.05 and 5 g/l, preferably 0.1 and 3.5 g/l.

In a preferred embodiment at the beginning of step a) the amount of carbon source is 5 to 50 g/l, preferably 10 to 50 g/l. The values depend on the molar mass of the carbon source and may be adapted to the content of C needed.

In a preferred embodiment a content of 5 to 30 g/l C, 0.1 to 4 g/l N and 0.1 to 3.5 g/l P is preferred.

In a preferred embodiment the bacteria are to this solution to obtain an inoculum.

The bacteria may also be added in an inoculum to this mixture. For the inoculum a content of 5 to 30 g/l C, 0.1 to 1.5 g/l N and 0.2 to 5 g/l P is preferred.

The values for the beginning of step a) refer to the values after adding the inoculum.

The preferred sources for C, N and/or P for inoculum are the same as mentioned for medium for step a).

In a preferred embodiment at the beginning of step a) the following amounts are present:
Carbon source (glycerol, sucrose or glucose or fructose) 15 to 70 g/l, (NH₄)₂SO₄ 1 to 5 g/l, KH₂PO₄ 0.5 to 20 g/l, Citric acid × 1 H₂O 0.1-3 g/l and NaH₂PO₄ 0 to 2 g/l.

Further salts of Mg or Ca may be present. Additionally, a trace element solution is added, comprising Zn, Mn, B, Co, Cu, Ni and Mo.

In a preferred embodiment at the beginning of step a) the reactor is filled at a volume of 5 to 20 % of its total volume.

Step a) is preferably run at a temperature between 20 and 40 °C, more preferably at a temperature between 29 to 35 °C.

It may be necessary to stir the reactor.

During the growth of the bacteria the growth nutrients present are consumed. In a preferred embodiment at least some nutrients are fed into the medium in order to keep these nutrients preferably in the ranges as mentioned above.

The feeding may add to the volume of the medium inside the reactor. Preferably no medium is removed during cultivation.

In a preferred embodiment the feeding solution is added at a rate between 0.1 to 5 % /h calculated from the total volume of the reactor, preferably 0.2 to 1 %. The feeding rate can be adapted based on the content of the feed and/or the cultivation conditions, especially the pressure used.

The feeding solution preferably at least comprises at least one carbon source.

In another embodiment the feeding solution comprises at least one carbon source, at least one nitrogen source.

In another embodiment the feeding solution comprises at least one carbon source, at least one nitrogen source and at least one phosphor source. The preferred sources are the same as mentioned for medium for step a)

In another embodiment the carbon source is identical with the carbon source of the starting conditions.

In a preferred embodiment the feeding solution comprises a carbon content of 100 to 500 g/l, preferably 150 to 300 g/l.

In an embodiment of the invention the feeding solution further comprises a content of N of 1 to 10 g/l, preferably 1 to 5 g/l.

In an embodiment of the invention the feeding solution further comprises a content of P of 0.5 to 15 g/l, preferably 1 to 10 g/l.

Preferably the feed comprises the content of C or C, N and P as previously mentioned or each in their preferred values.

The feed solution may comprise further salts of Ca and /or Mg, as well as acids like citric acid. The feed may also comprise a trace element solution.

In a preferred embodiment the feed comprises 150 to 300 g/l C.

The preferred sources for C, N and/or P for the feed are the same as mentioned for medium for step a)

In a preferred embodiment the feed comprises the following ingredients:
Carbon source (Glucose or sucrose or glycerol) 150 to 300 g/l of C.

In a preferred embodiment the feed only comprises a carbon source.

The growth of the bacteria is continued until a cell density of at least 10 (measured by OD at 600 nm), preferably at least 20, more preferably at least 30 is reached. The relevant growth point may also be related to other measurements, like time or composition.

Step a) is usually run for at least 5 hours up to 50 hours, preferably 20 hours up to 35 hours.

In the next step the bacteria are grown under autotrophic conditions, except that additionally at least one precursor of further monomers is added before and/or during this step. Under such conditions CO₂ and the carbon source are used as carbon source for the bacteria.

These conditions can be seen as mixotrophic, since more than one carbon source is used.

The amount of carbon source and the precursor added in such a total amount so that the polymer produced will contain carbon from CO₂ and the added carbon source and further monomers derived from the precursors. By this at least a part of the polymer produced will bind the CO₂ from the used atmosphere.

In a preferred embodiment the carbon source and precursor are added to an amount for up to 90 mol.-% of the carbon atoms of the polymer produced. These values are the calculated values, this means that if the polymer produced comprises 100 moles of carbon atoms, only 90 moles of carbon source is added during the process. In an even more preferred embodiment, the amount is between 5 mol.% and 90 mol.-%, even more preferred 5 mol.-% and 70 mol.-%, even more preferred 10 mol.-% and 50 mol.-%. In a preferred embodiment the carbon source is added in an amount of 10 mol.-% to 40 mol.-%.

In this step the media is contacted with an atmosphere comprising H₂, CO₂ and optional O₂. In order to minimize the risk of explosion the content of O₂ is less than 10 % (v/v). In a preferred embodiment the content of CO₂ is between 2 % and 40 % (v/v). In a preferred embodiment the content of H₂ is 50 % to 92 % (v/v).

In a preferred embodiment the content of O₂ is less than 8 % (v/v), preferably less than 6 % (v/v), even more preferably less than 5 % (v/v), especially preferred less than 3.1 % (v/v).

In a preferred embodiment the content of O₂ is less than 8 % (v/v), CO₂ is between 2 % and 40 % (v/v) and H₂ is between 50 % and 92 % (v/v), preferably the content of O₂ is less than 4 % (v/v), CO₂ is between 4 % and 40 % (v/v) and H₂ is between 50 % and 92 % (v/v).

Surprisingly it has been found that the addition of at least a precursor in the second step allows an efficient production of PHB-copolymers with further modifications. It also allows the use of less H₂ in the process.

In a preferred embodiment the amount of H₂ is between 50 % (v/v) and 85 % (v/v), even more preferred between 50 % and 82 % (v/v).

In a preferred embodiment the amount of H₂ is between 50 % (v/v) and 85 % (v/v), while the amount of CO₂ is more than 10 % (v/v), even more preferred the amount of H₂ is between 50 % and 82 % (v/v) while the amount of CO₂ is more than 15 % (v/v). The values adapt if O₂ is also present in the system in the amounts mentioned above.

In a preferred embodiment of the invention the amount of CO₂ is above 10 % (v/v), while the amount of H₂ is less than 85 % (v/v), preferably the amount of CO₂ is above 15 % (v/v), while the amount of H₂ is less than 82 % (v/v).

In a preferred embodiment of the invention the amount of H₂ is between 50 % (v/v) and 85 % (v/v), while the amount of CO₂ is from 10 % to 50 % (v/v), even more preferred the amount of H₂ is between 50 % and 82 % (v/v) while the amount of CO₂ is from 15 % to 50 % (v/v), preferably 15 % to 45 % (v/v). The values adapt if O₂ is also present in the system in the amounts mentioned above. Preferably the range of CO₂ is adapted based on the amount of O₂ present. O₂ is present preferably in an amount less than 5 % (v/v), preferably less than 3.1 % (v/v). Such an amount is sufficiently low to increase the safety of the process.

In a preferred embodiment the content of nitrogen in the atmosphere is less than 1 % (v/v) if present. Some minor amounts of nitrogen may be carried into the reaction vessel by not degassing the solutions fed into the reactor.

The source for such an atmosphere may be synthesis gas.

If precursors of further monomers are added the amount of CO₂ is preferably between 1 and 40 % (v/v), preferably 2 % and 30 % (v/v), preferably in the same ranges as described previously.

The ratios mentioned are the ratios present at the beginning of step b). Since the gases are used during the fermentation it may be necessary to adjust the amounts to the previous ranges during the fermentation. In a more preferred embodiment, the ratios are kept within these ranges during step b).

The pressure in step b) is at least 1 barg or gauge pressure. In a preferred embodiment the pressure is at least 2 barg, more preferably at least 3 barg.

In a preferred embodiment the pressure ranges from 2 to 20 barg, preferably 3 to 20 barg, more preferably 3 to 10 barg. The pressure is measured under cultivation conditions.

The pressure is the preferably kept in these ranges during step b). In a preferred embodiment the pressure during whole step b) is at least 1 barg.

Based on the standard atmospheric pressure of 1.013 bar 1 barg as used in the present application corresponds to 2.013 bar absolute pressure. All other ranges are adapted accordingly.

By using increased pressure, the growth of PHA-copolymer is expedited and the duration of step b) is shortened. Surprisingly the increased pressure also led to PHA-copolymer with different properties compared to PHA-copolymer obtained without increased pressure.

Preferably the content of the different gases is measured by the partial pressures.

Preferably the pressure is kept constant for the duration of step b). More preferably the atmosphere composition and pressure are kept constant for the duration of the step b).

In another embodiment of the invention at least one carbon source is fed into the system in step b). By this the amount of H₂ needed can be reduced, preferably the amount of H₂ and O₂ needed can be reduced.

The carbon source can also be combined with carbon sources as described previously. This may be sugars like sucrose, fructose or glucose, polyols like glycerol, organic acids or salts or esters thereof, as acetic acid or malate or ethyl acetate.

In order to produce copolymers precursors for further monomers are added in step b). These are typically salts of organic acids, preferably sodium or potassium salts. Examples for such salts are the corresponding salts of propanoic acid, butanoic acid, pentanoic acid or hexanoic acid, depending on the length of the side chain needed. These salts are not as good as the precursors of the present invention. The carbon source is preferably soluble in the medium.

It was now found that by adding a specific type of precursor a wider range of copolymers can be produced. Those precursors are better soluble and/or less toxic for the bacteria.

The at least one precursor is selected from an organic substance comprising at least 3 carbon atoms and at least 2 oxygen atoms, wherein the organic substance in its hydrolyzed form comprises at least two of carbon atoms are part of a functional group selected from COOH, C=O, C-OH. The at least one precursor is preferably soluble in the medium. In another embodiment these are salts of organic acids, preferably sodium or potassium salts. The carbon atoms are part of a functional group if they are the carbon atom in the corresponding COOH, C=O or C-OH groups.

In a preferred embodiment two of the carbon atoms in the hydrolyzed form are part of a functional group selected from COOH, C=O or C-OH.

The hydrolyzed form is the form of the substance, in which all ether or ester bonds are hydrolyzed to a carboxylic acid group and a hydroxyl group, e.g. gamma butyrolactone corresponds to gamma hydroxobutyric acid or ether group into two hydroxyl groups. If the hydrolyzation results in more than one molecule, at least two carbon atoms with the functional groups COOH, C=O, C-OH are present in one of the molecules.

In a preferred embodiment the carbon atoms within the molecule, which are part of the functional group selected from COOH, C=O, C-OH are separated by at least one carbon atom not linked to oxygen, preferably by at least one CH₂-group.

In a preferred embodiment the molecular mass of the at least one precursor is below 400 g/mol, preferably below 300 g/mol, even more preferably below 200 g/mol.

In a preferred embodiment the organic substance comprises 3 to 12 carbon atoms, preferably 3 to 8 carbon atoms, even more preferably 3 to 6 carbon atoms.

The presence of the multiple polar groups increases the solubility of the substance so that the substance can be added in a much higher amount. This increases the inclusion of the resulting monomer in the PHA-copolymer.

In a preferred embodiment each of the organic substance is either a linear or cyclic molecule, preferably a linear molecule or a cyclic ester.

In a preferred embodiment each of the organic molecule in its hydroylzed form comprises a COOH group and a C-OH group, a COOH group and a COOH group, a C-OH group and a C-OH group, a COOH group and a C=O group, a C=O group and a C-OH group, a C=O group and a C=O group, preferably a COOH group and a C-OH group, a COOH group and a COOH group, a C-OH group and a C-OH group, a COOH group and a C=O group.

If the COOH group in the precursor is used as an ester it is preferably an alkyl ester with an alkyl group of 1 to 4 carbon atoms.

If the C-OH group in the precursor is used as an ether it is preferably an alkyl ether of the organic molecule with an alkyl group of 1 to 4 carbon atoms.

In preferred embodiment the organic substance corresponds already to the hydrolyzed form if it is not a cyclic ester. In this case the precursor is an organic substance already comprising the at least two carbon atoms, which are part of the functional group selected from COOH, C=O, C-OH, or the organic substance comprises an R-COOR-group in which the R groups form a ring, preferably a 4, 5, 6 or 7 membered ring.

Examples for such compounds are hydroxylated organic acids, which may also be used as ester or cyclized form, like Hydroxobutyricacid like beta-hydroxybutyric acid (cyclic ester is beta-butyrolactone), gamma-hydroxybutyric acid (cyclic ester is gamma-butyrolactone), valerolactones like beta-valerlolactones, gamma-valerlolactones, organic acids with Keto groups like pyruvic acid, acetoacetic adid, levulinic acid, Polyols like 1,4-butandiols, 1,4-pentanediol, 1,6-hexanediol, 1,3-propandiol, organic acids with at least two carboxylic acid groups like succinic acid.

Levulinic acids can be used to produce PHBV, but it can be added in much higher levels than propionic acid. It is less toxic for the bacteria than propionic acid or valeric acid.

Gamma-hydroxobutyric acid or gamma-butyrolactone and 1,4-butanediol can be used for approx. 50 mol-% 4B to produce PH3,4B.

The main difference between a carbon source and the precursor is that the precursor always forms the majority (more than 50 mol.-%) of the monomer of the produced polymer.

The precursors are chosen to produce a co-polymer depending on the carbon source used.

In a preferred embodiment only at least one precursor is fed in step b). In step b) no further nutrients are then fed into the reactor.

The addition of the precursor and/or carbon source can be in different point in time during and/or before step b). It is preferred that it is added after step a).

The precursor and/or carbon source can be added in a separate step before the pressure is applied. It is also possible that it is added during step b). It is also possible that the feeding starts before applying the atmosphere and ends during step b). The addition can be continuous or in one or more portions. It is important that the respective amount of carbon source is added in total.

The addition of the precursor and/or carbon source can be performed in a different reactor or the same reactor of the cultivating step. It is also possible that it is added to a different reactor, which is connected with the cultivating reactor.

In a preferred embodiment the precursor and/or carbon source is added at a rate between 0.1 to 5 % /h calculated from the total volume of the reactor, preferably 0.2 to 1 %. The feeding rate can be adapted based on the content of the feed and/or the cultivation conditions, especially the pressure used.

In preferred embodiment each of the at least one precursor is added in an amount of 0.1 to 15 g/g % (weight % precursors / main carbon source), preferably 0.1 to 10 g/g%. The main carbon source is the carbon source added in the feed in step a) or the combined carbon source added in step a) and b). The content of seed culture is not taken into account. The amount is the amount in g as dissolved in the added feed.

Preferably at least 80 mol % of the amount of each precursors added is included into the PHA-copolymer.

In a preferred embodiment one precursor or a mixture of 2, 3 or 4 precursors is used. The precursors can each be added in different amount to adjust the properties of the resulting copolymer. It is also possible to use different precursors leading to the same monomer in the final PBH-copolymer.

In a preferred embodiment at least one precursor is chosen from levulinic acid, succinic acid, gamma-butyrolactone or 1,4-butanediol.

A preferred combination of precursors is levulinic acid and gamma-butyrolactone or levulinic acid and succinic acid.

The pH in step b) is preferably 6.5 to 7.5, more preferably 6.8 to 7.0. The pH may be adjusted using acid and/or bases, preferably sulfuric acid and/or ammonium hydroxide.

As a medium the same medium as step a) may be used, but without any nitrogen source.

In step b) the cultivation is preferably running under at least nitrogen deficient conditions. The nitrogen source limits the biomass accumulation. This leads to PHA accumulation.

In a preferred embodiment the cell dispersion at the end of step a) is directly used for step b) as starting medium.

The temperature in step b) is preferably between 20 °C and 45 °C, more preferably between 25 °C and 35 °C.

It may be necessary to stir the reactor during the reaction.

The reaction in step b) is running until the amount of PHA is formed, usually until a content of 50 % to 90 % of PHA by weight calculated from the dry weight of the whole biomass is formed.

Under these conditions it is possible to run step b) until final cell densities of more than 50 g/L, preferably more than 100 g/L is reached.

The reaction is also preferably stopped before the Mw of the PHA and/or PHB starts decreasing due to side reactions.

The duration of the cultivation is usually 10 to 100 hours, preferably 20 to 80 hours, more preferably 20 to 60 hours.

The reaction may also be stopped if a certain OD and/or cell dry weight (CDW) is reached. A preferred OD is an OD of more than 100, preferably more than 200.

Step b) may be run in the same or a different reactor than step a), preferably a different reactor.

If necessary further purification steps, like filtration or centrifugation are performed between the two steps.

In a preferred embodiment the cells are separated from the medium before the next purification steps.

It is also possible to wash the cells with an alcohol like methanol and/or ethanol.

The PHA-copolymer is intracellular, i.e. formed inside the bacteria. For the extraction several methods are possible.

In an embodiment of the invention the polymer is extracted from the cells using a solvent for the Polymer, preferably a polar organic solvent, more preferably acetone, chloroform, dimethyl carbonate or propylene carbonate, especially acetone or dimethyl carbonate. Also, a mixture of solvents may be used. It may be necessary to induce cell lysis before or during extraction. This can be achieved for example by mechanical stress and/or induced by the solvent for the polymer.

In an embodiment the cells are first broken by mechanical stress, e.g. increased atmosphere pressure. The polymer is then extracted using a solvent for the polymer, preferably a polar organic solvent, more preferably acetone, chloroform, dimethyl carbonate or propylene carbonate, especially acetone or dimethyl carbonate. Also, a mixture of solvents may be used.

In a preferred embodiment a further solvent with a higher boiling point than the solvent for polymer is also added. For example, an oil or an alkane with 10 to 14 carbon atoms. The further solvent is preferably used in a ratio of 1:20 to 1:4 by weight compared to the solvent for the polymer.

If from this mixture the solvent for the polymer is removed the polymer can be recovered as flakes or powder of high purity. Preferably the solvent for polymer is removed by heat. The solvent for polymer may be reused for a further extraction.

It is also possible that the cell lysis is combined with the extraction step, since acetone also leads to cell lysis. In this embodiment the acetone and optionally the further solvent is added to the separated cells. The amount of solvent for PHA-copolymers, especially acetone, and further solvent is preferably used in an excess compared to the weight of the separated cells. Preferably in an amount of at least 2 times the weight, more preferably at least 5 times the weight of the separated cells.

It is also possible that the cell lysis is separated from the extraction step, by using a homogenizer for cell lysis. In this embodiment the dimethyl carbonate and optionally the further solvent is added to the separated cells. The amount of solvent for PHA, especially dimethyl carbonate, and further solvent is preferably used in an excess compared to the weight of the separated cells. Preferably in an amount of at least 2 times the weight, more preferably at least 5 times the weight of the separated cells.

In a preferred embodiment solvent for PHA-copolymer and the further solvent is added and the mixture is then mixed and the non-aqueous phase is separated.

The PHA-copolymer produced is obtained as flakes during the removal of the solvent for the polymer, especially acetone or dimethyl carbonate.

The PHA-copolymer produced by the present process has a narrow molecular weight distribution and a very high content of the one or more comonomers.

Another object of the invention is a PHA-copolymer produced by the process of the present invention, preferably comprising at least three different monomers.

Another object of the invention is a PHA-copolymer comprising at least three different monomers chosen from 3HB, 3HV and 4HB. Such a copolymer can be produced using levulinic acid and gamma-butyrolactone as further precursors added.

The polymer can be used for various products depending on its properties. It may also be blended with other polymers.

Another object of the invention is a moulded article, granulate or a master batch comprising or formed from a PHA-copolymer as described previously.

The moulded articles can thereby be produced in any way, for example by extrusion, casting, injection moulding, pressing, sintering, calendering, film-blowing, melt-spinning, compression moulding and/or thermoforming, for components in automobile construction, transport and/or communications, components for industrial equipment, machine- and plant construction, household appliances, containers, devices for medical technology, components for electrics or electronics. Hence, the invention likewise relates to the use of a polymer material according to the invention for the previously mentioned purposes.

The polymer may be used for the production of coating materials, foils, films, laminates, fibers, moulded parts, moulded articles, injection moulded articles, e.g. bottles or fibers, extrudates, containers, packaging materials, coating materials, particles, beads, micro beads and medicine dispensers.

Preferably the coating material may be produced by spray drying, more preferably by spray drying directly from the polymer solution. The polymer solution is sprayed over the materials to be coated at the evaporation temperature of the solvent of the polymer solution in order to coat the material forming a solid polymer coat on the surface.

Preferably the fibers may be produced by spinning, more preferably by spinning directly from the polymer solution.

The polymer can be formed to any product as known from the previous products. It is also possible to add usual additives and other polymers.

### Examples

NaH₂PO₄ is used as dihydrate.

### Comparative Example A

A seed culture medium A was prepared with Glucose 15 g/l, (NH₄)₂SO₄ 1 g/l, MgSO₄× 4H₂O 0.2g/l g/l, KH₂PO₄ 1.5 g/l, Na₂HPO₄ 3.55 g/l, yeast extract 1 g/l and Trace elements solution 10 ml/l (ZnSO₄ × 7 H₂O 2.26 g, MnSO₄ × 1 H₂O 0.34 g, CaCl₂ × 2H₂O 2 g, CuSO₄ × 5 H₂O 1 g, FeSO₄ × 7 H₂O 10 g, (NH₄)₆Mo₇O₂₄ × 4 H₂O 0.106 g, Na₂B₄O₇ 0.122 g, HCl 37% 10 ml and distilled water 1000.00 ml).

To obtain the inoculum the bacteria (*Cupriavidus necator* H16) is added to the seed culture medium and the inoculum is added to a reactor.

Under growing conditions, a feed solution is added to the reactor. For chemolithotropic growth, the following feed solution was used comprising Glucose 500 g/l, without any co-substrate.

This solution was fed to the reactor under stirring at a rate of 3 - 5 ml/h until an OD of 50 was reached. Usually, this is before a reaction time of 28-30 h.

For autolithotropic growth the reaction mixture stays in the same reactor.

For autolithotropic growth CO₂, H₂ and O₂ are fed to the reactor with a total pressure of 3 barg (H₂: 80%, 2.4 barg; CO₂: 17 %, 0.5 barg; O₂: 3%, 0.1 barg) .

The reactor is stirred and the fermentation is run until an OD >200 is reached. Usually, the reaction time is at least 50 hours, e.g. after 68 hours an CDW of 65.9 g/L (cell dry weight) is reacted in the present example.

The fermentation is then stopped and the PHA is extracted.

### Example 1B

A seed culture medium B was prepared with Sucrose 20 g/l, (NH₄)₂SO₄ 2 g/l, MgSO₄× 4H₂O 1.0 g/l, KH₂PO₄ 0.6 g/l, Citric acid × 1 H₂O 0.11 g/l, NaH₂PO₄ 1.43 g/l, CaCl₂ × 2 H₂O 0.1 g/l and Trace elements solution 3 ml/l (ZnSO₄ × 7 H₂O 0.10 g, MnCl₂ × 4 H₂O 0.03 g, H₃BO₃ 0.30 g, CoCl₂ × 6 H₂O 0.20 g, CuCl₂ × 2 H₂O 0.01 g, NiCl₂ × 6 H₂O 0.02 g, Na₂MoO₄ × 2 H₂O 0.03 g and distilled water 1000.00 ml).

The seed culture medium was inoculated with bacteria (Cu*priavidus necator* H16) and the inoculum is added to a reactor.

Under growing conditions, a feed solution is added to the reactor. For chemolithotropic growth the following feed solution was used comprising Glucose 500 g/l.

This solution was fed to the reactor under stirring at a rate of 3 - 5 ml/h until an OD of 50 was reached. Usually, this is before a reaction time of 28-30 h.

For autolithotropic growth CO₂, H₂ and O₂ is fed to the reactor with a total pressure of 3.1 barg (H₂: 81%, 2.5 barg; CO₂: 16 %, 0.5 barg; O₂: 3%, 0.1 barg). Levulinic acid to an amount of 3 g/g % in total is added stepwise.

The reactor is stirred and the fermentation is run until an OD >200 is reached. Usually, the reaction time is at least 50 hours, e.g. after 54 hours and CDW of 80.5 g/l is reacted in the present example. This leads to 64.9 g/l product. From these cells, PHBV is obtained. (Elastic modulus 0.95 GPa, Tm = 167 °C, Content of V approx. 6%, Eta= 4.3 g/l).

### Example 1C

A seed culture medium C was prepared with Glycerol 50 g/l, (NH₄)₂SO₄ 4 g/l, MgSO₄× 4H₂O 1.2 g/l, KH₂PO₄ 13.3 g/l, Citric acid × 1 H₂O 1.85 g/l and Trace elements solution 10 ml/l (ZnSO₄ × 7 H₂O 0.10 g, MnCl₂ × 4 H₂O 0.03 g, H₃BO₃ 0.30 g, CoCl₂ × 6 H₂O 0.20 g, CuCl₂ × 2 H₂O 0.01 g, NiCl₂ × 6 H₂O 0.02 g, Na₂MoO₄ × 2 H₂O 0.03 g and distilled water 1000.00 ml).

To the seed culture medium the bacteria (*Cupriavidus necator* H16) is added to obtain the inoculum and the inoculum is added to a reactor.

Under growing conditions, a feed solution is added to the reactor. For chemolithotropic growth the following feed solution was used comprising Glucose 500 g/l.

This solution was fed to the reactor under stirring at a rate of 3 - 5 ml/h until an OD of 50 was reached. Usually, this is before a reaction time of 28-30 h.

For autolithotropic growth CO₂, H₂, and O₂ are fed to the reactor with a total pressure of 3.1 barg (H₂: 80.6%, 2.5 barg; CO₂: 16.1 %, 0.5 barg; O₂: 3.2%, 0.1 barg) . Levulinic acid of 6 g/g % is added stepwise.

The reactor is stirred and the fermentation is run until an OD >200 is reached. Usually, the reaction time is at least 50 hours. e.g. after 54 hours and CDW of 80.5 g/l is reacted in the present example. This leads to 65.4 g/l product. From these cells, PHBV is obtained.

### Example 1D

A seed culture medium C was prepared with Glycerol 50 g/l, (NH₄)₂SO₄ 4 g/l, MgSO₄× 4H₂O 1.2 g/l, KH₂PO₄ 13.3 g/l, Citric acid × 1 H₂O 1.85 g/l and Trace elements solution 10 ml/l (ZnSO₄ × 7 H₂O 0.10 g, MnCl₂ × 4 H₂O 0.03 g, H₃BO₃ 0.30 g, CoCl₂ × 6 H₂O 0.20 g, CuCl₂ × 2 H₂O 0.01 g, NiCl₂ × 6 H₂O 0.02 g, Na₂MoO₄ × 2 H₂O 0.03 g and distilled water 1000.00 ml).

To the seed culture medium the bacteria (*Cupriavidus necator* H16) is added to obtain the inoculum and the inoculum is added to a reactor.

Under growing conditions, a feed solution is added to the reactor. For chemolithotropic growth the following feed solution was used comprising Glucose 500 g/l.

This solution was fed to the reactor under stirring at a rate of 3 - 5 ml/h until an OD of 50 was reached. Usually, this is before a reaction time of 28-30 h.

For autolithotropic growth CO₂, H₂, and O₂ are fed to the reactor with a total pressure of 3.1 barg (H₂: 80.6%, 2.5 barg; CO₂: 16.1 %, 0.5 barg; O₂: 3.2%, 0.1 barg) . Levulinic acid of 4 g/g % and 1,4 butanediol 1.5 g/g % is added stepwise.

The reactor is stirred and the fermentation is run until an OD >200 is reached. Usually, the reaction time is at least 50 hours. e.g. after 54 hours and CDW of 80.5 g/l is reacted in the present example. This leads to 61.7 g/l product. From these cells, PHBV is obtained.

### Example 1E

A seed culture medium C was prepared with Glycerol 50 g/l, (NH₄)₂SO₄ 4 g/l, MgSO₄× 4H₂O 1.2 g/l, KH₂PO₄ 13.3 g/l, Citric acid × 1 H₂O 1.85 g/l and Trace elements solution 10 ml/l (ZnSO₄ × 7 H₂O 0.10 g, MnCl₂ × 4 H₂O 0.03 g, H₃BO₃ 0.30 g, CoCl₂ × 6 H₂O 0.20 g, CuCl₂ × 2 H₂O 0.01 g, NiCl₂ × 6 H₂O 0.02 g, Na₂MoO₄ × 2 H₂O 0.03 g and distilled water 1000.00 ml).

To the seed culture medium the bacteria (*Cupriavidus necator* H16) is added to obtain the inoculum and the inoculum is added to a reactor.

Under growing conditions, a feed solution is added to the reactor. For chemolithotropic growth the following feed solution was used comprising Glucose 500 g/l.

This solution was fed to the reactor under stirring at a rate of 3 - 5 ml/h until an OD of 50 was reached. Usually, this is before a reaction time of 28-30 h.

For autolithotropic growth CO₂, H₂, and O₂ are fed to the reactor with a total pressure of 3.1 barg (H₂: 80.6%, 2.5 barg; CO₂: 16.1 %, 0.5 barg; O₂: 3.2%, 0.1 barg) . Levulinic acid of 4 g/g % and gamma-butyrolactone 0.5 g/g % is added stepwise (g/g% are weight % precursors / main carbon source).
The reactor is stirred and the fermentation is run until an OD >200 is reached. Usually, the reaction time is at least 50 hours. e.g. after 54 hours and CDW of 80.5 g/l is reacted in the present example. This leads to 61.7 g/l product. From these cells, PH3,4HBV (poly-3HB-co-3HV-co-4HB) is obtained.

### Example 1F

A seed culture medium C was prepared with Glycerol 50 g/l, (NH₄)₂SO₄ 4 g/l, MgSO₄× 4H₂O 1.2 g/l, KH₂PO₄ 13.3 g/l, Citric acid × 1 H₂O 1.85 g/l and Trace elements solution 10 ml/l (ZnSO₄ × 7 H₂O 0.10 g, MnCl₂ × 4 H₂O 0.03 g, H₃BO₃ 0.30 g, CoCl₂ × 6 H₂O 0.20 g, CuCl₂ × 2 H₂O 0.01 g, NiCl₂ × 6 H₂O 0.02 g, Na₂MoO₄ × 2 H₂O 0.03 g and distilled water 1000.00 ml).

To the seed culture medium the bacteria (*Cupriavidus necator* H16) is added to obtain the inoculum and the inoculum is added to a reactor.

Under growing conditions, a feed solution is added to the reactor. For chemolithotropic growth the following feed solution was used comprising Glucose 500 g/l.

This solution was fed to the reactor under stirring at a rate of 3 - 5 ml/h until an OD of 50 was reached. Usually, this is before a reaction time of 28-30 h.

For autolithotropic growth CO₂, H₂, and O₂ are fed to the reactor with a total pressure of 3.1 barg (H₂: 80.6%, 2.5 barg; CO₂: 16.1 %, 0.5 barg; O₂: 3.2%, 0.1 barg). Levulinic acid of 4 g/g % and succinic acid of 0.7 g/g % is added stepwise (g/g% are weight % precursors / main carbon source).
The reactor is stirred and the fermentation is run until an OD >200 is reached. Usually, the reaction time is at least 50 hours, e.g. after 54 hours and CDW of 80.5 g/l is reacted in the present example. This leads to 61.7 g/l product. From these cells, PH3,4HBV (poly-3HB-co-3HV-co-4HB) is obtained.

### Comparative 1D

A seed culture medium A was prepared with Glucose 20 g/l, (NH₄)₂SO₄ 4 g/l, MgSO₄× 4H₂O 1.2 g/l, KH₂PO₄ 4 g/l, Citric acid × 1 H₂O 1.86 g/l and Trace elements solution 10 ml/l (ZnSO₄ × 7 H₂O 0.10 g, MnCl₂ × 4 H₂O 0.03 g, H₃BO₃ 0.30 g, CoCl₂ × 6 H₂O 0.20 g, CuCl₂ × 2 H₂O 0.01 g, NiCl₂ × 6 H₂O 0.02 g, Na₂MoO₄ × 2 H₂O 0.03 g and distilled water 1000.00 ml).

To obtain the inoculum the bacteria (*Cupriavidus necator* H16) is added to the seed culture medium and the inoculum is added to a reactor.

Under growing conditions, a feed solution is added to the reactor. For chemolithotropic growth the following feed solution was used comprising Glucose 660 g/l, (NH₄)₂SO₄ 12 g/l, NaH₂PO₄ 12 g/l, MgSO₄ × 7H₂O 3.6 g/l, KH₂PO₄ 12 g/l, Citric acid 30 g/l, Trace element solution 15 ml/l.

This solution was fed to the reactor under stirring at a rate of 3 - 5 ml/h until an OD of 20 is reached. Usually this is before a reaction time of 21 h.

For autolithotropic growth the reaction mixture is either put into a new reactor or stays in the same reactor.

For autolithotropic growth CO₂, H₂ and O₂ is fed to the reactor at atmospheric pressure with composition of the gas mixture H₂: 84%, CO₂: 13 %, O₂: 3%.

The reactor is stirred and the fermentation is run until an OD >200 is reached. Usually, the reaction time is at least 150 hours, e.g. after 184 hours and OD of 230g/l is reacted in the present example.

The fermentation is then stopped and the PHA is extracted with Dimethylcarbonate as solvent.

The properties of the different polymers are shown in table 1:

**Table 1:**

| Property | PHB Comparative 1 A | PHBV Example 1B | PHBV Example 1C | PH3, 4HBV Example 1D | PHB Comparative 1D |
|---|---|---|---|---|---|
| Density [g/cm³] (v1183) | 1.25 | 1.25 | 1.25 | 1.25 | 1.25 |
| Molecular weight [g/mol] ( GPC) | 745 k | 652.3 k | 728 k | 713.6 k | 571 k |
| Melting point [°C] (11357-3) | 178 | 164 | 162 | 145 | 179 |
| Crystallinity % (NMR) | 67 | 71 | 70 | 69 | 75 |

Although the melting temperature is the same as expected for pure PHB, the modulus and the tensile properties are more similar to those of the PHBH copolymers.

### DSC measurements

Mettler DSC 30 scanner was used. The tests were conducted with a controlled flow of nitrogen. The samples were subjected to the following thermal cycle: two heating steps from -100 °C to 200 °C interspersed by a cooling step from 200 °C to -100 °C. The heating/cooling rate was 10 °C/min.

From the DSC thermograms the melting point (Tₘ₁) of the polymer, the crystallization temperature (T_{c}) under cooling conditions and the melting point in the second heating scan (Tₘ₂) were identified. The integration of the peaks allowed to estimate the melting enthalpy under the first (ΔHₘ₁) and the second (ΔHₘ₂) heating scans and under cooling (ΔH_{c}) conditions.

### Molecular weight

The molecular weight was measured indirectly, by intrinsic viscosity, where relation between the viscosity and the molecular weight is given by the Mark-Houwink expression (a = 0.78, k = 0.000118).

### Uniaxial tensile tests

The test was performed using an Instron tensile tester model 4250 equipped with a 100 N load cell. The test was carried out at a cross-head speed equal to 1 mm/min. The specimens for the test have been prepared by cutting a film of the studied PHB. The film was obtained from the dissolution of the polymer with chloroform into a Petri dish and the subsequent evaporation of the solvent. Five specimens were tested.

### Nuclear magnetic resonance (NMR)

All the samples were analyzed with solid-state nuclear magnetic resonance spectroscopy (Bruker 400Avance WB equipped with double channel 4mm CPMAS probe, Bruker Spa, Rheinstetten) at ¹³C frequency of 100.46 MHz with both simple pulse and cross-polarized sequences at room temperature at 8 kHz of spinning speed to avoid signal overlap. All the results of profile fitting are considered acceptable with a confidence level >97%.

In the NMR spectrum both methyl and methylene signals are represented by sharp peaks together with a right broad shoulder. Thus, these shoulders are proof of a different chain packing in the solid state. The presence of this type of shoulder in the case of other polymers is usually attributed to an amorphous component.

## Claims

1. Method for producing PHA-copolymers comprising the following steps:
a) growing bacteria under heterotrophic conditions in a media;
b) cultivating the bacteria under autotrophic conditions under an atmosphere of CO₂, H₂ and optional O₂, wherein the amount of O₂ is below 10 % (v/v) and pressure is at least 1 barg, wherein at least one precursor for further monomers is added before and/or during step b), wherein the at least one precursor is selected from an organic substance comprising at least 3 carbon atoms and at least 2 oxygen atoms, wherein the organic substance in its hydrolyzed form comprises at least two of carbon atoms are part of a functional group selected from COOH, C=O, C-OH.

2. Method according to claim 1, wherein the bacterium is a wild type bacterium.

3. Method according to one of the claims 1 or 2, wherein the bacterium is *Cupriavidus necator.*

4. Method according to one of the claims 1 to 3, wherein the organic substance comprises 4 to 12 carbon atoms.

5. Method according to one of the claims 1 to 4, wherein in step a) the bacteria are grown under exponential growth conditions.

6. Method according to one of the claims 1 to 5, wherein the pressure in step b) is at least 2 barg.

7. Method according to one of the claims 1 to 6, wherein the pressure in step b) ranges from 2 to 20 barg.

8. Method according to one of the claims 1 to 7, wherein content of CO₂ in step b) is between 2 % and 90 % (v/v).

9. Method according to one of the claims 1 to 8, wherein the content of H₂ in step b) is between 50 % and 92 % (v/v).

10. PHA-copolymer as produced by the process according to one of the claims 1 to 9.

11. PHA-copolymer comprising at least three different monomers.

12. Moulded article, granulate or master batch comprising the PHA-copolymer according to claim 10 or 11.

13. Use of the PHA-copolymer according to claim 10 or 11 for the production of coating materials, foils, films, laminates, fibers, moulded parts, moulded articles, injection moulded articles, extrudates, containers, packaging materials, coating materials, particles, beads, micro beads and medicine dispensers.

14. Method for producing a coating material using a polymer solution of PHA-copolymer according to claim 10 or 11.

15. Method for producing fibers using a polymer solution of PHA-copolymer according to claim 10 or 11.
